# EUROPÄISCHE PATENTSCHRIFT

(11) **EP 1 242 056 B1**
(45) Veröffentlichungstag und Bekanntmachung des Hinweises auf die Patenterteilung: **01.03.2006**
(21) Anmeldenummer: 00979325.8
(22) Anmeldetag: 19.12.2000
(51) Int. Cl.: A61K 9/48, A61J 3/07, C08K 5/06

(54) **ZUM SCHMIEREN VON STRÄNGEN, INSBESONDERE BÄNDERN**
USE OF A WATER-SOLUBLE AGENT CONTAINING POLYGLYCEROLS FOR LUBRICATING EXTRUSIONS, IN PARTICULAR STRIPS
UTILISATION D'UN AGENT HYDROSOLUBLE CONTENANT DE LA POLYGLYCERINE POUR LUBRIFIER DES BANDES, EN PARTICULIER DES RUBANS

(30) Priorität: 30.12.1999 EP 99811218
(43) Veröffentlichungstag der Anmeldung: 25.09.2002
(73) Patentinhaber: Greither, Peter, 9533 Kirchberg (CH)
(72) Erfinder: BROCKER, Erich, CH-9533 Kirchberg (CH); MAIER, Hans-Jürgen, CH-8636 Wald-Oberholz (CH)
(74) Vertreter: Wenger, René
(86) Internationale Anmeldenummer: PCT/CH2000/000670
(87) Internationale Veröffentlichungsnummer: WO 2001/049271

(56) Entgegenhaltungen:
- EP-B- 0 676 945
- DE-C- 651 060
- FR-A- 863 405
- US-A- 3 637 774
- US-A- 5 362 564
- PATENT ABSTRACTS OF JAPAN vol. 12, no. 197 (M-706), 8. Juni 1988 (1988-06-08) & JP 63 002615 A (SODEITSUKU), 7. Januar 1988 (1988-01-07)

## Beschreibung

Gegenstand der vorliegenden Erfindung ist die Verwendung eines mindestens ein Polyglycerin enthaltenden wasserlöslichen Mittels zum Schmieren von Strängen, insbesondere Bändern aus einem biopolymeren Stoff in einem wenigstens die Förderung der Stränge beinhaltenden Arbeitsprozess, ein Verfahren zur Herstellung einzeldosierter Darreichungsformen und die Wiederverwendung des mit einem solchen Schmiermittels vermischten Abfalls eines solchen Arbeitsprozesses gemäss den unabhängigen Patentansprüchen 1, 7, 10 und 13.

Weichkapseln sind ideal als Verpackung für Öle, für lipophile Suspensionen sowie für auf hydrophilen Polymeren basierenden Suspensionen. Insbesondere ermöglichen sie einzeldosierte Darreichungsformen. Die Anwendungen sind vielfältig und umfassen die Gebiete Technik, Nahrung, Gesundheit und Kosmetik. Die effizienteste Herstellung von Weichkapseln erfolgt nach dem Rotary-Die-Verfahren, das in US 2 288 327 erstmals beschrieben wurde. Die Ausformung der Hülle, deren Befüllung sowie das Verschliessen der Kapsel erfolgen in einem Arbeitsgang an der Kapselfüllstation durch Verschweissen zweier Endlosbänder aus Gelatine oder einem anderen Biopolymer zwischen zwei als Stanzwerkzeugen rotierenden Formwalzen (vergleiche Bauer, K., ,Die Herstellung von Hart- und Weichgelatinekapseln' in "Die Kapsel", Hrsg. Fahrig, W. et al., Wissenschaftliche Verlagsgesellschaft mbH Stuttgart 1983).

Das Rotary-Die-Verfahren ist aufgrund der damit produzierbaren hohen Stückzahlen das führende Verfahren und ist in seinen Grundzügen im wesentlichen unverändert geblieben; das gilt auch für daraus abgeleitete, ähnliche Verfahren (Norton, Accogel).

Einer dieser Grundzüge ist, dass als Schmiermittel für die spannungsfreie Zuführung der Bänder zur Kapselfüllstation Mineralöle oder Fette (Triglyceride) verwendet werden.

Das klassische Rotary-Die-Verfahren erlaubt in einem ununterbrochenen, einzigen Produktionsschritt die Herstellung von bis zu 100'000 Kapseln pro Stunde. Eine als Sol bei ca. 60°C flüssig gehaltene Gelatinemasse mit den darin enthaltenden Hilfsstoffen wird über spezielle Giessvorrichtungen auf zwei Kühltrommeln zu endlosen, gummiartig elastischen Bändern von ca. 0,8 mm Dicke geformt und dann über (geschmierte) Umlenk- und Zwischenrollen zu zwei rotierenden Formwalzen geleitet. Auf den Kühltrommeln vollzieht sich durch Unterschreiten der Gelierungstemperatur von ca. 40°C der Phasenübergang vom Sol zum Gel. Die beiden Gelatinebänder werden in dem schmalen Spalt zwischen den zwei gegenläufig rotierenden Formwalzen zusammengefügt; hier vollzieht sich der Einfüll- und Verschliessvorgang. Die Formwalzen dienen dem Verschweissen der Gelatinebänder und dem Ausstanzen der fertigen, gefüllten Kapsel. Oberhalb bzw. fast zwischen den Formwalzen arbeitet der Füllkeil, über den das Füllgut in die Kapseln gelangt und dessen eingebaute Thermoelemente die Siegelwärme für das Verschweissen der beiden Gelatinebänder entlang des Kapselumrisses generieren. Auch andere thermisch verformbare Materialbänder lassen sich problemlos mit dem Rotary-Die-Werkzeug zu Kapseln verarbeiten.

Das zur Schmierung der Bandzuführung beidseitig auf die Bänder aufgebrachte Mineralöl oder Triglycerid verhindert ein Kleben an Maschinenteilen, reduziert die Reibung und dient gleichzeitig als Dichtmittel für eine satte Auflage des Füllkeils im Spalt zwischen den beiden Bändern. Ein luftdichter Abschluss ist entscheidend für das Einpumpen des Füllgutes unter leichtem Druck, wobei die Kapsel ihre eigentliche Form annimmt. Gleichzeitig überträgt das Schmiermittel die Siegelwärme vom Füllkeil auf die Oberfläche der Bänder und damit auf die zu verschweissenden Oberflächen; der Füllkeil erzeugt eine Temperatur von über 40°C, sodass das Verschweissen der Kapselhälften bewirkt wird. Das Schmieren eines Gelatinefilms ist beispielsweise in der EP-A-676 945 beschrieben.

Ein Schmiermittel muss also über einen grösseren Temperaturbereich gute Gleiteigenschaften bzw. geeignete Viskositäten aufweisen. Zudem darf das Schmiermittel nicht in das Band diffundieren und dort oder an der Kapseloberfläche unerwünschte Veränderungen hervorrufen.

Als geeignete Schmiermittel wurden bisher Mineralöle oder mittelkettige Triglyceride verwendet. Aus der hydrophoben Natur dieser Schmiermittel leiten sich aber auch Nachteile ab, die bisher bei der Herstellung von Kapseln zwangsläufig in Kauf genommen wurden: Auf der Kapselinnenseite anhaftendes Öl beeinträchtigt die feste Verschweissung der Kapselhälften und führt gelegentlich zu Füllgutleckagen. Die Schmiermittel beeinträchtigen die Weiterverarbeitung, insbesondere die Trocknung der frischen Kapseln, die besonders bei Gelatine zur Senkung des Wassergehaltes durchgeführt wird. Der dünne Schmiermittelfilm, insbesondere Ölfilm, auf der Aussenseite führt zum Verkleben der Kapseln während der Trocknung.

Der Schmiermittelfilm muss daher in einem zusätzlichen Arbeitsschritt entfernt werden. Verwendet werden vor allem Waschbäder mit organischen Lösungsmitteln wie z.B. Methylenchlorid, 1,1,1-Trichlorethan, 1,1,2-Trichlorethylen, Naphta, Isopar, Freone, etc. Der Einsatz von z.T. toxischen, umweltschädigenden, leichtflüchtigen organischen Lösungsmitteln ist jedoch unerwünscht oder gar unzulässig. Die Entfernung des Schmiermittelfilms mit diesen Lösungsmitteln führt zu hohen Kosten für die Rückstandsanalytik im Endprodukt und regulatorischen Schwierigkeiten (Einsatzverbot, Umweltauflagen und nicht eliminierbare Kontamination des Lösungsmittels durch die aktiven Stoffe der Darreichungsform bei der Wiederaufbereitung). Vor allem ist aber der zusätzliche Arbeitsschritt des Waschens unerwünscht, da der damit verbundene Aufwand proportional mit der produzierten Menge ansteigt.

Ein weiterer Nachteil der Verwendung dieser im Stand der Technik bekannten Schmiermittel liegt in der Kontamination der sogenannten Netzabfälle, d.h. der nach Ausstanzen der Kapseln überbleibenden perforierten Bänder, durch das Schmiermittel. Die Netzabfälle betragen etwa 30 - 60% der ursprünglich eingesetzten Masse. Die Netzabfälle können bisher nicht im selben oder ähnlichen Verfahren wiederverwertet werden. Die Zusammensetzung der Masse wird durch enthaltende Spuren von Öl, das nicht vollständig abgetrennt werden kann, nachteilig verändert. Beim Einschmelzen ergibt sich ein öliges zwei-Phasen-Gemisch, das nicht wiederverwertbar ist.

Die Menge der Netzabfälle, d.h. des als Ausschuss verworfenen Materials, erhöht wesentlich die Produktionskosten. Zusätzlich kostensteigend ist der besondere Aufwand mit dem der Netzabfall entsorgt wird. Die Wiederverwendung der Netzabfälle im Produktionsprozess oder ähnlicher Produkte, bei denen ebenfalls Spuren von Mineralöle oder Triglyceriden nicht tolerierbar sind, ist daher aus Kostenerwägungen sehr wünschenswert.

Alternativverfahren zur Herstellung von Weichkapseln, wie das "Drop and Blow-Verfahren", erreichen nicht die Produktionsleistung des Rotary-Die-Prozesses oder sind nicht gleich robust in der Prozessführung, was sich z.B. im Einstellen der Temperatur und Viskosität der Masse, oder der mangelnden Verarbeitung von hochviskosen Füllgütern oder Pasten negativ bemerkbar macht.

Aufgabe der vorliegenden Erfindung ist es, die Nachteile des Bekannten zu vermeiden. Insbesondere besteht die Aufgabe darin ein Schmiermittel bereitzustellen, das die Wiederverwendung damit kontaminierter Netzabfälle erlaubt. Weiterhin besteht die Aufgabe darin, einen abschliessenden Waschschritt überflüssig zu machen oder gegebenenfalls ohne Einsatz von organischen Lösungsmitteln das Schmiermittel von der Kapseloberfläche zu entfernen. Es ist eine weitere Aufgabe der vorliegenden Erfindung die Verschweissung der Kapselhälften nicht zu beeinträchtigen. Weiterhin soll ein Verfahren zur Verfügung gestellt werden, mit dem die Netzabfälle wiederverwendet und zu weiteren Produkten geformt werden können.

Diese Aufgaben werden gelöst durch die Merkmale der unabhängigen Ansprüche.

Insbesondere werden sie gelöst durch die Verwendung eines mindestens ein Polyglycerin enthaltenden wasserlöslichen Mittels zum Schmieren von Strängen aus mindestens einem biopolymeren Stoff in einem wenigstens die Forderung der Stränge beinhaltenden Arbeitsprozess. Insbesondere soll der Arbeitsprozess Bestandteil des Prozesses zur Herstellung von Weichkapseln sein.

Es wurde überraschend gefunden, dass hydrophile Polyglycerine als Schmiermittel bei der Herstellung von Formkörpern, insbesondere Weichkapseln nach dem Rotary-Die-Verfahren, oder einem anderen, ähnlichen Verfahren zur Herstellung von Formkörpern aus einem Biopolymer geeignet sind.

In der EP-A-676 945 wird vorgeschlagen, bei der Herstellung von Kapseln mit dem Rotary-Die-Verfahren eine derart geringe Menge eines herkömmlichen Schmiermittels einzusetzen, dass auf dem fertigen Produkt nur noch geringe Mengen dieses Schmiermittels vorhanden sind. Dies macht die Bereitstellung von Dosiervorrichtungen für das Schmiermittel erforderlich.

In der US-3 637 774 wird allgemein die Möglichkeit beschrieben, Polyglycerine auch als Schmiermittel einzusetzen.

In der DE-C-651060 ist die Verwendung von Polyglycerinen zum Schmieren von Vorrichtungsteilen, die mit fett-, öl- oder mineralöllösenden Stoffen in Berührung kommen, auch in Kombination mit sirupösen Verdickungsmitteln wie wässrigen Abkochungen von Pflanzenschleimen beschrieben.

Das Mittel zum Schmieren ist wasserlöslich bzw. wasserdispergierbar und umfasst ein oder mehrere Polyglycerine, vorzugsweise aus 2 bis 28 Monomereinheiten. Während viele hydrophile Stoffe wie beispielsweise Glycerin, Polyethylenglycole oder Propylenglycole als Schmiermittel ungeeignet sind, da sie entweder ungenügend schmieren, ungenügend dichten oder eine unerwünschte Veränderung der Gelmatrix bewirken, verbinden Polyglycerine als Schmiermittel sehr gute Gleiteigenschaften mit einem neutralem Verhalten insbesondere auch gegenüber einer wasserhaltigen Matrix wie z.B. bei Gelatine. Es ist möglich, nebst Gelatine auch andere zu Bändern oder Strängen verarbeitete Biopolymere, z.B. Stärke, Stärkederivate, Cellulose, Cellulosederivate, Galactomannane, Gummiarten, Agar-Agar, und andere sowie Mischungen dieser Biopolymere zu verwenden. Insbesondere nach einem neuartigen Verfahren hergestellte Stärkebänder können eingesetzt weden. Die Bänder werden dabei nicht gegossen wie bei der Gelatine, sondern extrudiert. Das Material kann unter wesentlich höheren Temperaturen verarbeitet werden als Gelatine und hat einen weit geringeren Wasseranteil. Eine Schmierung der Bänder zwischen Extruder und Rotary-Die-Werkzeug ist aber ebenfalls erforderlich. Das neuartige Stärkematerial ist in der Europäischen Patentanmeldung 99811071 = EP-A-1103254 (Veröffentlichungstag : 30.06.2001) beschrieben, deren Offenbarung hiermit in die vorliegende Anmeldung übernommen wird.

Das äusserlich auf die Kapseln oder andere Formkörper aufgebrachte Polyglycerin kann durch Wasser oder physiologisch unbedenkliche Stoffe wie Ethanol leicht entfernt werden. Bei Verwendung des erfindungsgemässen Schmiermittels ist der Waschschritt, d.h. die Entfernung des Schmiermittels von der Formkörperhülle, nur noch fakultativ. Das Abwaschen des Films vor oder während der Trocknung ist nicht mehr unbedingt erforderlich. Die Polyglycerine als hydrophile Substanzen können bei geeigneter Kettenlänge von der Oberfläche der Matrix absorbiert werden bzw. diffundieren in die Matrix wenigstens oberflächlich. Eine Molekülgrösse des Polyglycerins von 2 bis 10 Monomereinheiten ist besonders geeignet. Mit einem dünnen Film von Polyglycerin bedeckte Kapseln verkleben während oder Trocknung nicht, vermutlich in Folge der besseren Benetzung der Kapseloberfläche bzw. der Oberflächenspannung, was unnötige Produktionsverluste vermeidet. Insbesondere dem auf die Kapselbefüllung nachfolgenden Trocknungsvorgang kommt zugute, dass das leicht hygroskopische Polyol Wasser aus der Kapselhülle zieht und generell dem Trocknungsprozess keine hydrophobe Diffusionsbarriere entgegensetzt.

Die Polyglycerine zeigen überragende Eigenschaften als Gleitmittel, dichten im Bereich des warmen Füllkeils gut ab und fördern die thermomechanische Verschweissung der Kapselhälften. Insbesondere bei hydrophilen Füllgütern, bei denen die Wärmeübertragung durch ein konventionelles, hydrophobes Schmiermittel zu ungenügender Schweissnahtbildung und somit zu Leckagen führen kann, ist ein hydrophiles Schmiermittel auf Polyglycerinbasis überlegen.

Ein weiterer Vorteil bei der Verwendung von Polyglycerin in Schmiermitteln besteht darin, dass die oberflächlich mit Schmiermittel überzogenen Netzabfälle, bei der Herstellung von Formkörpern, insbesondere Weichkapseln einer Wiederverwendung zugeführt werden können. Ein hydrophiles Schmiermittel bildet beim Einschmelzen eine homogene Phase, die für eine Wiederverwertung z.B. durchaus im gleichen Prozess, zur Verfügung steht. Anhaftende Reste von Füllgut sind tolerierbar, wenn der Netzabfall zu Formkörpern recycliert wird, der mit dem gleichen Füllgut befüllt wird.

Wie z.B. aus der US-A-5,362,564 ersichtlich ist, wird Polyglycerin als Weichmacher beispielsweise in Gelatinefilmen bereits eingesetzt. Insofern die thermischen oder mechanischen Eigenschaften der recyclierten Netzabfälle, beispielsweise von Gelatine, durch die Polyglycerine überhaupt eine Veränderung erfahren, so erfolgt diese Veränderung in vorhersagbarer und erwünschter Weise. Das durch die Recyclierung der Netzabfälle eingebrachte Polyglycerin bewegt sich mengenmässig maximal bei einigen Prozent bzw. kann so eingestellt werden. Durch Beimischen eines Teils frischer Biopolymermasse mit geringerem Weichmacheranteil, der sich auch aus anderen Weichmachern als Polyglycerin zusammensetzen kann, wird dieser Effekt zudem neutralisiert. Auch können Rakels eingesetzt werden, um die an den Netzabfällen anhafteten Reste von Polyglycerin durch Abstreifen unter den Bereich von ca. 1% zu senken.

Ein Strang oder Band auch im Sinne der vorliegenden Erfindung, wird durch Giessen, Blasen, Extrudieren, Tropf- bzw. Tauchverfahren hergestellt. Die Biopolymermasse kann dabei z.B. durch Gel-Sol-Übergang die notwendige Viskosität bzw. Festigkeit annehmen oder bereits vorher besessen haben (z.B. bei extrudierbaren Mischungen).

Polyglycerine im Sinne der vorliegenden Erfindung sind Oligomere mit höchstens 28 Monomereinheiten Glycerin HO-CH₂-CH(OH)-CH₂-OH, die z.B. in einer Kondensationsreaktion aus Glycerol unter Ausbildung eines Polyethers erhalten werden. Herstellungstechnisch fallen die Oligomeren dabei in Gemischen unterschiedlicher Kettenlängen an. Deshalb bedeutet die Angabe eines bestimmten Oligomeres, z.B. Decaglycerin eine Mischung, welche mehrheitlich dieses bestimmte Oligomer enthält. So können auch noch Anteile an Di-, Tri- und weiteren Glycerinen vorhanden sein.

Es sind aber auch andere Synthesewege möglich, z.B. die Umsetzung eines Startmoleküls Glycerin mit dem entsprechenden (Hydroxy-Methyl)-Oxiran. Die Kondensation von Glycerol führt mit zunehmender Zahl verknüpfter Monomere zu einer wachsenden Anzahl beliebig verzweigter Konstitutionsisomere aufgrund der dreifachen Funktionalität des Glycerolmonomers. Es ist auch möglich, die Kondensationspolymerisation mit einem partiell durch Schutzgruppen derivatisierten Glycerin durchzuführen, wobei die Monomeren nur bifunktionell sind und sich unverzweigte Polyether ergeben.

Polyglycerine im Sinne der vorliegenden Erfindung sind durch ihre Molmasse charakterisiert, die der Zahl der darin miteinander über Etherbindung verknüpften Glycerolmonomeren entspricht. (Lexikon der Hilfsstoffe, P. Fiedler, Editio Cantor Verlag Aulendorf, 1996). Während das Monomere Glycerin rein oder als Mischung mit anderen organischen Stoffen nur mässig gut als Gleitmittel geeignet ist, sind dessen höhere Homologen vom Diglycerin an als Schmiermittel im Rotary-Die-Verfahren oder einem ähnlichen Arbeitsprozess (s.o.) gut geeignet. Polyglycerine im Sinne der vorliegenden Erfindung entstehen durch Verknüpfung von 2 bis 28 Glycerolmonomeren. Vom Glycerin unterscheiden sie sich durch eine mit der Kettenlänge zunehmende Viskosität und Solvatationsvermögen. Ein Polyglycerine im Sinne der vorliegenden Erfindung ist weiterhin bei Raumtemperatur eine Flüssigkeit und ist bei Raumtemperatur mit Wasser in jedem Verhältnis mischbar. Polyglycerine mit mehrheitlich Oligomeren eines Molekulargewichts von 750 g/mol ist noch flüssig. Eine bevorzugte Ausführungsform im Sinne der vorliegenden Erfindung ist Decaglycerin.

Solange die Eigenschaft als Schmiermittel bzw. die Wasserlöslichkeit im Sinne des vorher Gesagten nicht beeinträchtigt wird, können die Polyglycerine auch partiell derivatisiert, z.B. verestert sein. Es können auch Gemische von Polyglycerinen und Polyglycerinderivaten, insbesondere Polyglycerinester als Schmiermittel eingesetzt werden.

Ein weiterer Vorzug des erfindungsgemässen Schmiermittels ergibt sich dann, wenn eine Kapselnachbehandlung erfolgt, bei der durch Filme oder Überzüge spezielle Eigenschaften wie Zeit- oder pHkontrollierte Löslichkeit im Magen erreicht werden sollen. Für diese Anwendungen werden wasserbasierte Überzüge bevorzugt, um ein Handling mit leichtflüchtigen organischen Lösungsmitteln zu vermeiden. Reste von Polyglycerin z. B. auf der Kapseloberfläche sind mit derartigen Überzügen kompatibel. Es ist auch möglich, derartige Überzüge bereits dem Schmiermittel beizumischen, z.B. wässrige Mischungen von Polyglycerin und Hydroxymethylcellulose oder anderer Stärke- und Cellulosederivate, soweit sich dadurch nicht die Schmiermitteleigenschaften wesentlich verändern. In einer weiteren bevorzugten Ausführungsform umfasst das Schmiermittel deshalb ein Cellulosederivat, vorzugsweise ein als magensaftresistenter Überzug geeignetes Cellulosederivat. Ein Beispiel für ein magensaftresistenten Überzug aus einem Cellulosederivat ist z.B. Hydroxy-propylmethyl-cellulose-acetatsuccinat (HPMCAS). HPMCAS ist in wässriger Lösung nur bei einem pH > 5 löslich.

In einer bevorzugten Ausführungsform enthält das Schmiermittel zusätzlich strukturverändernde Substanzen für das biopolymere Material, z.B. Xylose für Gelatine.

Bei Zugabe von Hydroxymethylpropylcellulosephtalat, Cellulosephtalat, Emulsionen aus Methylmethacrylat ist es auch möglich für den nachfolgenden Lackauftrag einen Primer zu schaffen.

Ein Schmiermittel im Sinne der vorliegenden Erfindung ist wasserlöslich und umfasst, mit Wasser oder Polyglycerin homogen mischbare organische Stoffe, in Polyglycerin oder Wasser, optional unter Verwendung eines Emulgators, gelöste organische Stoffe, vorzugsweise Cellulosederivate, oder mit Wasser homogen mischbare organische Lösungsmittel, allein oder in beliebiger Kombination.

Die mit Wasser oder polyglycerin mischbaren organische stoffe umfassen aliphatische oder cyclische Alkohole, Glycol, Glycerin, Propylenglycol oder Polypropylenglycol, Ethylenglycol oder ein Polyethylenglycol alleine oder in Kombination. Die Beimischung dieser hydrophilen Substanzen erlaubt es, die Eigenschaft eines auf Polyglycerin basierenden Schmiermittels optimal auf die Eigenschaften des verwendeten Biopolymers oder auf die spezifischen Produktionsanforderungen abzustimmen. Die Vorteile der hydrophilen Polyglycerine als Schmiermittel werden dabei gewahrt. Insbesondere Komponenten wie Polyalkenylglycole diffundieren bei hinreichender Kettenlänge nicht in die Gelmatrix.

Biopolymere wie Cellulosederivate können als zusätzliche, rheologieeinstellende Additive dienen. In der Galenik sind vielfach wasserlösliche, durch Veretherung mit (Hydroxy-Alkoxygruppen oder durch Veresterung z.B. mit Phthalsäure derivatisierte Cellulosen gebräuchlich.

Vorzugsweise beträgt der Mischungsanteil des Polyglycerins am Schmiermittel mindestens 5% , in einer bevorzugten Ausführungsform mindestens 15 %, und in einer am meisten bevorzugten Ausführungsform mindestens 40% des Gesamtgewichts.

In einer weiteren bevorzugten Ausführungsform besteht das Polyglycerin oder Polyglyceringemisch des erfindungsgemässen Schmiermittels aus unverzweigten Kettenmolekülen. Derartige Kettenmoleküle zeigen besonders gute Gleitmitteleigenschaften in einem weiten Temperaturbereich zwischen ca. 5 bis 50°C und verbinden diese aufgrund leichter Strukturviskosität mit guten Dichtungseigenschaften im Bereich des Füllkeils. Die Viskosität steigt linear mit der Kettenlänge an; die Zugabe oder alleinige Verwendung von unverzweigten Polyglycerine zu den erfindungsgemässen Schmiermitteln erlauben damit insbesondere in Mischung mit den anderen, oben genannten Stoffen die gezielte Herstellung eines Schmiermittels geeigneter Viskosität.

Ein weiterer Gegenstand der vorliegenden Erfindung ist ein Verfahren zur Herstellung von Dosierkapseln unter Verwendung eines ein Polyglycerin oder Polyglyceringemisch mit 2 - 28 Monomereinheiten, vorzugsweise 2 - 10 Monomereinheiten, enthaltenden wasserlöslichen Schmiermittels gemäss dem oben Gesagten in einem die Förderung.eines Stranges, vorzugsweise eines Bandes, eines Biopolymers beinhaltendem Arbeitsprozess, umfassend die Schritte des Erzeugens wenigstens eines kontinuierlichen ersten Stranges und wenigstens eines kontinuierlichen zweiten Stranges, des Auftragens eines Schmiermittels auf wenigstens eine Seite wenigstens eines Stranges, des Vereinigens des ersten und des zweiten Stranges zu Formkörpern und des Einfüllens eines Füllgutes unter Ausbildung einer Kapselform und schliesslich die Trocknung der Kapseln.

Ein durch diese Schritte beschriebenes Herstellungsverfahren sind z.B. das Rotary-Die-Verfahren und das Accogel-Verfahren. Beide Verfahren erlauben die Herstellung von rundum verschlossenen Formkörpern, insbesondere Kapseln, aus einer Vielzahl von filmbildenden Biopolymermischungen, wie z.B. Gelatine oder Stärke. Es ist aber auch möglich, derartige Kapseln im Reciprocating-Die-Verfahren (Norton) oder einem anderen, kontinuierlichen Stanzverfahren unter Verwendung von Gelbändern oder -strängen herzustellen.

Ein weiterer Vorteil des Verfahrens ist das Entfallen eines zusätzlichen Reinigungsschritts zum Entfernen des den Formkörper anhaftenden Schmiermittels. Das wasserlösliche Schmiermittel behindert die Trocknung nicht und verhindert das Verkleben der Kapseln. Trocknung im Sinne der vorliegenden Erfindung kann z.B. Umlufttrocknung sein, es kann sich aber auch um Trocknung z.B. in Gegenwart eines Trockenmittels bei Unterdruck u.ä. handeln. Die Trocknung dient in jedem Falle der Senkung des Lösungsmittelgehalts der Gelmatrix, vorzugsweise des Wassergehaltes; der Formkörper wird dadurch verfestigt.

Erfindungsgemässe Biopolymermischungen enthalten üblicherweise Weichmacher, z.B. Glycerin, Polyglycerin, Sorbitol, Propylglycol, Ditmethylpolysiloxane und Ethylenglycol. Es ist möglich, filmbildende Mischungen zu verwenden, die aus Gelatine, Cellulose oder Cellulosederivat, Stärke oder einem anderen Biopolymer, Mischungen daraus oder wenigstens zum Teil aus einem Derivat oder Abbauprodukt eines Biopolymers bestehen.

Es ist auch möglich, die Kapseln vor oder während der Trocknung von den auf der Kapselhülle verbliebenen Resten des Schmiermittels zu reinigen. Vorteil dabei ist, das keine organischen Lösungsmittel verwendet werden müssen. Das Schmiermittel ist von der Oberfläche der Kapseln durch unschädliche, hydrophile Flüssigkeiten wie z.B. (kaltes) Wasser, Alkohole, ein Gemisch dieser Stoffe, oder aber durch ein festes Bindemittel z.B. Baumwolltücher leicht zu entfernen. Derartige Methoden zur Entfernung des auf der Kapseloberfläche zurückbleibenden dünnen Films des Schmiermittels sind Gegenstand bevorzugter Ausführungsformen des erfindungsgemässen Verfahrens.

Ein weiterer Gegenstand der vorliegenden Erfindung ist die wenigstens teilweise Verwendung der bei der Herstellung von Weichkapseln in einem kontinuierlichen Verfahren, nach Ausstanzen der Kapseln aus einem oder mehreren mit Schmiermittel bestrichenen Bändern anfallenden Stanzabfälle als Ausgangsmaterial zur Herstellung von Bänder, wobei das Schmiermittel wasserlöslich oder wasserdispergierbar ist und ein oder mehrere Polyglycerine vorzugsweise mit 2 bis 28 Monomereinheiten umfasst.

Ein weiterer Gegenstand der vorliegenden Erfindung ist die Verwendung von biopolymeren Materialien enthaltenden ein wasserlösliches oder wasserdispergierbares Schmiermittel, welches ein oder mehrere Polyglycerine umfasst vorzugsweise mit 2 bis 28 Monomereinheiten, und als Abfall eines ersten Arbeitsprozesses zur Herstellung eines Formkörpers anfällt, in einem zweiten Arbeitsprozess.

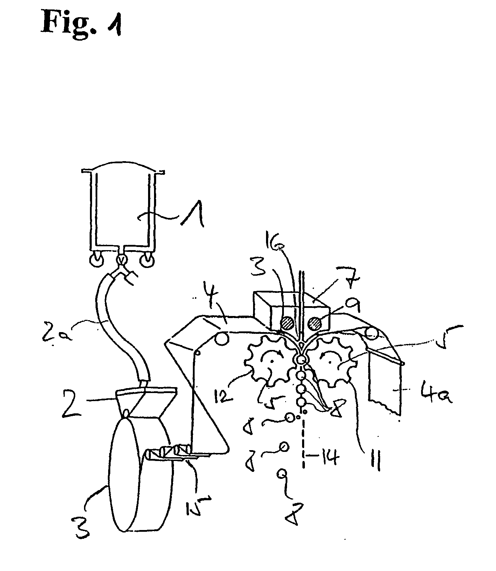

Figur 1 zeigt schematisiert das Rotary-Die-Verfahren.

Das Rotary-Die-Verfahren stützt sich auf eine Verkapselungsmaschine mit rotierenden Formwalzen 5,5a . Charakteristisch ist der einstufige Fertigungsgang. Die Herstellung der Kapselhülle, ihre Befüllung sowie das Ausstanzen der Kapseln erfolgen in einer einzigen Maschine, wie in der Figur 1 dargestellt.

Aus einem Gelatinevorratsbehälter 1 der das Gelatine-Sol mit Zuschlagstoffen wie z.B. Weichmachern bei 60 - 70 °C in flüssiger Form enthält, wird über eine spezielle Bandgiessvorrichtung 2 (mit Zuleitung 2a) auf einer Kühltrommeln 3 ein endloses gummiartig, elastisches Band von im Mittel ungefähr 0,8 mm Dicke gegossen. Auf der Kühltrommel 3, die in einem Temperaturbereich von 5 bis 25°C arbeitet, vollzieht sich der Übergang zum Gel.

Die genaue Kühltemperatur ist dabei ein wichtiger Verfahrensparameter, um die Eigenschaften des Gelbandes 4,4a d.h. die Elastizität zu steuern. Die Kühltrommel 3 verfügt über glatte Oberflächen, die überlicherweise nicht geschmiert werden. Das auf der Kühltrommel 3 erzeugte Gelatineband 4,4a wird dann über mehrere Schmierwalzen 15 und Umlenkwalzen spannungsfrei zu den Formwalzen 5,5a geführt. Die Schmierwalzen 15 verfügen z.B. über eine saugfähige Oberfläche und können von einem inneren Hohlraumvolumen mit Schmiermittel gespeist werden. Es sind aber auch andere Mittel für das Aufbringen des Schmiermittels auf das Gelatineband, wie Tauchen Sinterwalzen, etc. möglich.

Das Gelatineband 4 wird mit einer Geschwindigkeit von ungefähr 1 - 2 m/min transportiert, wobei einzelne Walzen mit Antriebsmitteln versehen sein können.

Oberhalb bzw. zwischen den Formwalzen 5,5a arbeitet der Füllkeil 7, über den das Füllgut 16, in die Kapseln 8 gelangt und dessen eingebaute Heizelemente 9 die Siegelwärme für das Verschweissen der aus den zwei Gelatinebändern 4, 4a gebildeten Kapselhälften abgeben. Aus den Gelatinebändern 4a,4 bilden die mit Aussparungen 12 versehenen, gegenläufigen Formwalzen 5, 5a durch Zusammenpressen flache, taschenartige Gebilde. Die Aussparungen 12 entsprechen dem Kapselumriss, entlang dem der Gelbänder 4, 4a verschweisst werden.

Kurz vor dem vollständigen Verschliessen der durch diese Aussparung 12 definierten Geltasche wird das Füllgut 16, durch den Füllkeil 7 in diese Taschen gedrückt, wobei die Kapsel 8 ihre eigentliche Form annimmt. Der Walzendruck auf die Ränder 11 der Aussparung 12 bewirkt ausserdem das Ausstanzen der Kapseln 8. Der Spalt 3 zwischen den zusammenlaufenden Gelatinebändern 4, 4a und dem Füllkeil 7 wird durch aufgestautes Schmiermittel abgedichtet. Diese Dichtungsfunktion ist essentiell für die korrekte Befüllung der Kapsel 8. Das Schmiermittel überträgt im Spalt 3 die Wärme der Heizelemente 9 vom Füllkeil 7 auf die Oberfläche der zu verschweissenden Gelatinebänder 4, 4a. Das Schmiermittel muss also in einem Temperaturbereich von 20°C (auf den Walzen 15) bis zur Heiztemperatur von ca. 40° (am Füllkeil 7) geeignete Schmier- eigenschaften aufweisen. Ein hydrophiles Schmiermittel interferiert weniger mit dem Verschweissen der feuchten Matrix der Gelatinebänder4, 4a. Das nach dem Ausstanzen der Kapseln verbleibende Gelatineband, das als Netzabfall 14 bezeichnet wird, ist auf der Oberfläche mit Schmiermittel kontaminiert. Konventionelle Schmiermittel (Mineralöle bzw. Triglyceride), erlauben die Wiederverwendung des Netzabfalls 14 durch Einschmelzen zum Sol nicht. Polyglycerine als.hydrophiles Schmiermittel mischen sich hingegen beim Einschmelzen homogen mit der Gelatinemasse und beeinflussen die Eigenschaften des daraus hergestellten Gelatinebandes 4, 4a nicht negativ bzw. in vorhersagbarer Weise und Ausmass, da Polyglycerin aus dem Stand der Technik als Weichmacher für Weichgelatinekapseln bekannt ist. Es ist z.B. möglich, die Gelatinemasse im Vorratsbehälter 1 teilweise aus Netzabfällen 14 und frischem Material mit geringerem Weichmacheranteil anzumischen. Dadurch machen sich die durch das Schmiermittel resultierenden Polyglycerinbeimengungen nicht störend bemerkbar.

### Vergleichsbeispiel 1

Ein erfindungsgemässes Schmiermittel wird hergestellt durch Mischen von drei Massenteilen Dekaglycerin (Hersteller: Sakamoto Yakuhin Kogyo C. Ltd.) mit einem Massenteil Wasser. Unter Verwendung diese Schmiermittels wurden Weichgelatinekapseln in einem Rotary-Die-Verfahren hergestellt. Die erhaltenen Kapseln zeigen eine sehr gut ausgebildete Naht, lassen sich schnell trocknen, zeigen Formstabilität und kleben nicht.

### Vergleichsbeispiel 2

Ein erfindungsgemässes Schmiermittel wird hergestellt durch Mischen von zwei Massenteilen Hexaglycerin mit zwei Massenteilen Wasser. Unter Verwendung dieses Bandgleitmittels wurden Weichgelatinekapseln im Rotary-Die-Verfahren hergestellt. Die erhaltenen Kapseln zeigen wiederum eine sehr gut ausgebildete Naht, lassen sich schnell trocknen und zeigen Formstabilität.

### Beispiel 3

Ein erfindungsgemässes Schmiermittel wird hergestellt von drei Massenteilen Hexaglycerin mit einem Massenteil PEG 400 (400 = Molekulargewicht). Unter Verwendung dieses Bandgleitmittels wurden Weichgelatinekapseln hergestellt nach dem Rotary-Die-Verfahren. Die erhaltenen Kapseln zeigen eine sehr gut ausgebildete Naht, lassen sich schnell trocknen und zeigen Formstabilität.

## Patentansprüche

1. Mittel zum Schmieren von Strängen aus mindestens einem biopolymeren Stoff in einem wenigstens die Förderung der Stränge beinhaltenden Arbeitsprozess, **dadurch gekennzeichnet dass** das Mittel zum Schmieren wasserlöslich ist und mindestens ein Polyglycerin oder mindestens ein partiell derivatisiertes Polyglycerin, vorzugsweise aus 2 bis 28 Monomereinheiten, sowie zusätzlich eine Komponente, ausgewählt aus der Gruppe bestehend aus mit Wasser oder Polyglycerin optional unter Verwendung eines Emulgators homogen mischbaren organischen Stoffen, in Polyglycerin oder Wasser gelösten organischen Stoffen, vorzugsweise Cellulosederivate, oder mit Wasser homogen mischbaren organischen Lösungsmitteln, allein oder in beliebiger Kombination, umfasst, wobei die mit Wasser oder Polyglycerin mischbaren organischen Stoffe aliphatische und/oder cyclische Alkohole, Glycol, Glycerin, Propylenglycol und/oder Polypropylenglycol, Ethylenglycol oder Polyethylenglycol, allein oder in Kombination, umfassen.

2. Mittel zum Schmieren gemäss Anspruch 1, **dadurch gekennzeichnet, dass** zusätzliche strukturverändernde Substanzen für den biopolymeren Stoff vorhanden sind.

3. Mittel zum Schmieren gemäss einem der vorhergehenden Ansprüche, **dadurch gekennzeichnet, dass** das Polyglycerin unverzweigt ist.

4. Mittel zum Schmieren gemäss einem der vorhergehenden Ansprüche, **dadurch gekennzeichnet, dass** das Schmiermittel wasserlöslich ist und mindestens ein Polyglycerin oder mindestens ein partiell derivatisiertes Polyglycerin aus 2 bis 28 Monomereinheiten und ein Cellulosederivat, vorzugsweise ein als magensaftresistenter Überzug geeignetes Cellulosederivat umfasst.

5. Mittel gemäss einem der vorhergehenden Ansprüche, **dadurch gekennzeichnet, dass** der Mischungsanteil des Polyglycerins oder Polyglycerinderivats am Schmiermittel mindestens 5% des Gesamtgewichts beträgt.

6. Mittel zum Schmieren nach einem der vorhergehenden Ansprüche, **dadurch gekennzeichnet, dass** der biopolymere Stoff Gelatine oder Stärke ist oder Gelatine oder Stärke enthält.

7. Verwendung von einem mindestens ein Polyglycerin oder mindestens ein partiell derivatisiertes Polyglycerin, vorzugsweise aus 2 bis 28 Monomereinheiten, enthaltenden wasserlöslichen Mittels zum Schmieren von Strängen aus mindestens einem biopolymeren Stoff in einem wenigstens die Förderung der Stränge beinhaltenden Arbeitsprozess.

8. Verwendung nach Anspruch 7, **dadurch gekennzeichnet, dass** das Mittel zum Schmieren von Bändern aus mindestens einem biopolymeren Stoff in einem Prozess zum Herstellen von Kapseln eingesetzt wird.

9. Verwendung eines Mittels zum Schmieren gemäss Anspruch 4 oder 5 für die Herstellung einer vorzugsweise magensaftresistent überzogenen Kapsel.

10. Verfahren zur Herstellung von Dosierkapseln unter Verwendung eines mindestens ein Polyglycerin oder mindestens ein partiell derivatisiertes Polyglycerin mit 2 bis 28 Monomereneinheiten, vorzugsweise 4 bis 10 Monomereneinheiten, enthaltenden wasserlöslichen Schmiermittels in einem die Förderung eines Stranges aus einem Biopolymer beinhaltenden Arbeitsprozess, umfassend die Schritte
- Erzeugen wenigstens eines kontinuierlichen ersten Stranges und wenigstens eines kontinuierlichen zweiten Stranges;
- Auftragen des Schmiermittels auf wenigstens eine Seite wenigstens eines Stranges;
- Vereinigen des ersten und des zweiten Stranges zu Formkörpern und Einfüllen eines Füllgutes unter Ausbildung einer Kapselformen;
- Trocknung der Kapseln.

11. Verfahren gemäss Anspruch 10, **dadurch gekennzeichnet, dass** in einem zusätzlichen Verfahrensschritt vor der Trocknung das Entfernen des Schmiermittels durch Wasser, einen Alkohol oder ein Gemisch dieser Stoffe vorgenommen wird.

12. Verfahren gemäss Anspruch 10, **dadurch gekennzeichnet, dass** in einem zusätzlichen Verfahrensschritt vor oder während der Trocknung das Entfernen des Schmiermittels durch ein festes Bindemittel, vorzugsweise Baumwollgewebe, vorgenommen wird.

13. Verwendung der ein biopolymeres Material enthaltenden und mit einem wasserlöslichem Schmiermittel umfassend mindestens ein Polyglycerin oder mindestens ein partiell derivatisiertes Polyglycerin aus 2 bis 28 Monomereinheiten vermischten Abfälle eines ersten Arbeitsprozesses zur Herstellung eines Erzeugnisses, vorzugsweise eines Formkörpers, in einem zweiten Arbeitsprozess.

14. Verwendung nach Anspruch 13, **dadurch gekennzeichnet, dass** es sich um Stanzabfälle handelt, die in einem kontinuierlichen Verfahren zur Herstellung von Weichkapseln nach Ausstanzen der Kapseln aus einem oder mehreren mit Schmiermittel beschichteten Bändern anfallen, zur Herstellung besagter Bänder.

## Revendications

1. Agent de lubrification de bandes se composant d'au moins une substance biopolymère au cours d'un processus de travail comprenant au moins le transport des bandes, **caractérisé en ce qu'**il est soluble dans l'eau et **en ce qu'**il comprend au moins une polyglycérine ou au moins une polyglycérine partiellement transformée en un dérivé, composée, de préférence, de 2 à 28 unités de monomère, et en outre un composant sélectionné dans le groupe constitué par des substances organiques miscibles de manière homogène éventuellement en utilisant un émulsifiant, avec l'eau ou une polyglycérine, des substances organiques solubles dans l'eau ou une polyglycérine, de préférence des dérivés de cellulose, et des solvants organiques miscibles de façon homogène avec l'eau, seuls ou en une combinaison particulière, les substances organiques miscibles avec l'eau ou une polyglycérine comprenant des alcools aliphatiques et/ou cycliques, le glycol, la glycérine, le propylène glycol et/ou le polypropylène glycol, l'éthylène glycol ou le polyéthylène glycol, seuls ou en combinaison.

2. Agent de lubrification selon la revendication 1, **caractérisé en ce que** des substances complémentaires de modification de structure pour la substance biopolymère sont présentes.

3. Agent de lubrification selon l'une quelconque des revendications précédentes, **caractérisé en ce que** la polyglycérine n'est pas ramifiée.

4. Agent de lubrification, selon l'une quelconque des revendications précédentes, **caractérisé en ce qu'**il est soluble dans l'eau et **en ce qu'**il comprend au moins une polyglycérine ou au moins une polyglycérine partiellement transformée en un dérivé, composée de 2 à 28 unités de monomère et un dérivé de cellulose, de préférence un dérivé de cellulose approprié en tant que revêtement résistant au suc gastrique.

5. Agent selon l'une quelconque des revendications précédentes, **caractérisé en ce que** la proportion de mélange de la polyglycérine ou du dérivé de polyglycérine de l'agent de lubrification représente au moins 5 % du poids total.

6. Agent de lubrification, selon l'une quelconque des revendications précédentes, **caractérisé en ce que** la substance biopolymère est constituée par une gélatine ou un amidon ou contient une gélatine ou un amidon.

7. Utilisation d'un agent soluble dans l'eau contenant au moins une polyglycérine ou au moins une polyglycérine transformée partiellement en un dérivé, de préférence composée de 2 à 28 unités de monomère, pour la lubrification de bandes se composant d'au moins une substance biopolymère au cours d'un processus de travail comprenant au moins le transport des bandes.

8. Utilisation selon la revendication 7, **caractérisée en ce que** l'agent de lubrification des rubans se composant d'au moins une substance polymère est utilisé dans un procédé pour la fabrication de capsules.

9. Utilisation d'un agent de lubrification selon la revendication 4 ou 5 pour la fabrication d'une capsule pourvue d'un revêtement de préférence résistante au suc gastrique.

10. Procédé pour la fabrication de capsules de dosage avec utilisation d'un agent de lubrification soluble dans l'eau contenant au moins une polyglycérine ou au moins une polyglycérine transformée partiellement en un dérivé, composée de 2 à 28 unités de monomère, de préférence de 4 à 10 unités de monomère, dans un processus de travail comprenant le transport d'une bande se composant d'un biopolymère, comprenant les étapes :
- de production d'au moins une première bande continue et d'au moins une deuxième bande continue ;
- d'application de l'agent de lubrification sur au moins une face d'au moins une bande ;
- de réunion de la première et de la deuxième bandes en corps moulés et de remplissage avec un produit de remplissage en formant des capsules ;
- de séchage des capsules.

11. Procédé selon la revendication 10, **caractérisé en ce que** dans une étape de procédé supplémentaire, avant le séchage, on réalise l'élimination de l'agent de lubrification à l'aide d'eau, d'un alcool ou d'un mélange de ces substances.

12. Procédé selon la revendication 10, **caractérisé en ce que** dans une étape de procédé supplémentaire, avant ou durant le séchage, on réalise l'élimination de l'agent de lubrification à l'aide d'un liant solide, de préférence un tissu en coton.

13. Utilisation de déchets mélangés avec un agent de lubrification soluble dans l'eau, comprenant au moins une polyglycérine ou au moins une polyglycérine partiellement transformée en un dérivé, composée de 2 à 28 unités de monomère et comprenant un matériau biopolymère, d'un premier processus de travail, pour la fabrication d'un produit, de préférence d'un objet moulé, dans un deuxième processus de travail.

14. Utilisation selon la revendication 13, **caractérisée en ce qu'**il s'agit de déchets de découpe qui sont produits dans un procédé en continu pour la fabrication de capsules molles après la découpe des capsules dans un ou plusieurs rubans pourvu(s) d'un revêtement d'agent de lubrification pour la fabrication desdits rubans.

## Claims

1. An agent for lubricating strands of at least one biopolymeric substance in a working process including at least conveying of the strands, **characterised in that** the lubricating agent is water-soluble and includes at least one polyglycerol or at least one partially derivatised polyglycerol, preferably with 2 to 28 monomers units, and additionally a component selected from the group consisting of organic substances homogeneously miscible with water or polyglycerol optionally using an emulsifier, organic substances dissolved in polyglycerol or water, preferably cellulose derivatives, or organic solvents homogeneously miscible with water, alone or in any combination, wherein the organic substances miscible with water or polyglycerol include aliphatic and/or cyclic alcohols, glycol, glycerol, propylene glycol and/or polypropylene glycol, ethylene glycol or polyethylene glycol alone or in combination.

2. A lubricating agent according to claim 1 **characterised in that** there are additional structure-modifying substances for the biopolymeric substance.

3. A lubricating agent according to one of the preceding claims **characterised in that** the polyglycerol is unbranched.

4. A lubricating agent according to one of the preceding claims **characterised in that** the lubricant is water-soluble and includes at least one polyglycerol or at least one partially derivatised polyglycerol comprising 2 to 28 monomer units and a cellulose derivative, preferably a cellulose derivative suitable as a gastric acid-resistant coating.

5. An agent according to one of the preceding claims **characterised in that** the proportion of polyglycerol or polyglycerol derivative in the lubricant is at least 5% of the total weight.

6. A lubricating agent according to one of the preceding claims **characterised in that** the biopolymeric substance is gelatin or starch or contains gelatin or starch.

7. Use of a water-soluble agent containing at least one polyglycerol or at least one partially derivatised polyglycerol, preferably with from 2 to 28 monomer units, for lubricating strands of at least one biopolymeric substance in a working process including at least conveying of the strands.

8. Use according to claim 7 **characterised in that** the agent is used for lubricating strips of at least one biopolymeric substance in a process for the production of capsules.

9. Use of a lubricating agent according to claim 4 or claim 5 for the production of a preferably gastric acid-resistantly coated capsule.

10. A process for the production of dose capsules using a water-soluble lubricant containing at least one polyglycerol or at least one partially derivatised polyglycerol with 2 to 28 monomer units, preferably 4 to 10 monomer units, in a working process including conveying a strand of a biopolymer, including the steps:
- producing at least one continuous first strand and at least one continuous second strand;
- applying a lubricant to at least one side of at least one strand;
- combining the first and second strands to form shaped bodies and introducing a filling material with the formation of a capsule form; and
- drying the capsules.

11. A process according to claim 10 **characterised in that** in an additional process step prior to the drying operation removal of the lubricant is effected by water, an alcohol or a mixture of said substances.

12. A process according to claim 10 **characterised in that** in an additional process step prior to or during the drying operation removal of the lubricant is effected by a solid binding agent, preferably cotton cloth.

13. Use of the waste, containing a biopolymeric material and mixed with a water-soluble lubricant, including at least one polyglycerol or at least one partially derivatised polyglycerol with from 2 to 28 carbon atoms, from a first working process for the production of a product, preferably a shaped body, in a second working process.

14. Use according to claim 13 **characterised in that** the waste involves stamping waste which occurs in a continuous process for the production of soft capsules after stamping out the capsules from one or more strips coated with lubricant, for the production of said strips.
